# EUROPÄISCHE PATENTSCHRIFT

(11) **EP 3 715 456 B1**
(45) Veröffentlichungstag und Bekanntmachung des Hinweises auf die Patenterteilung: **27.08.2025**
(21) Anmeldenummer: 20164898.7
(22) Anmeldetag: 23.03.2020
(51) Int. Cl.: C12N 15/10

(54) **VERFAHREN ZUR ISOLIERUNG VON NUKLEINSÄUREN AUS PROBEMATERIALIEN**
METHOD FOR ISOLATING NUCLEIC ACIDS FROM SAMPLE MATERIALS
PROCÉDÉ D'ISOLATION DES ACIDES NUCLÉIQUES À PARTIR DE MATIÈRES D'ÉCHANTILLONNAGE

(30) Priorität: 28.03.2019 DE 102019108087
(43) Veröffentlichungstag der Anmeldung: 30.09.2020
(73) Patentinhaber: AXAGARIUS GmbH & Co. KG, 52355 Düren (DE)
(72) Erfinder: von Bohl, Andreas, 52072 Aachen (DE); Schüll, Yvonne, 52459 Inden Altdorf (DE)
(74) Vertreter: dompatent von Kreisler Selting Werner - Partnerschaft von Patent- und Rechtsanwälten mbB

(56) Entgegenhaltungen:
- EP-A1- 1 524 317
- EP-B1- 1 354 036
- US-A1- 2002 192 667
- US-A1- 2017 121 702

## Beschreibung

Die Erfindung ein Verfahren zur Isolierung von Nukleinsäuren wie DNA und RNA unter Verwendung einer nicht-alkoholischen und nicht-Chaotropsalz-basierenden Bindelösung, sowie die für solch ein Verfahren geeignete Bindelösungen und Kits.

### Hintergrund

Hintergrund der Erfindung ist die Entwicklung eines Verfahrens und eines Kits zur Isolierung von Nukleinsäuren (DNA/RNA) aus sogenannten "Hard-to-Lyse"-Probenmaterialien (Mikroorganismen wie Bakterien und Pilze, Insekten, fetthaltige Gewebe) und Gewebsproben mittels magnetischer Partikel.

Die Isolierung von Nukleinsäuren (DNA/RNA) mittels Chaotropsalz, Alkohol, vorzugsweise Ethanol und Isopropanol und einer festen Phase ist nach wie vor nahe am Stand der Technik und wird mehrfach seit vielen Jahren eingesetzt. Die Anbindung der Nukleinsäure, zum Beispiel an magnetische Partikel, erfolgt in der Regel nach dem enzymatischen oder mechanischen Aufschluss der biologischen Probe mittels Zugabe von Chaotropsalz und/oder organischen Lösemitteln, vorzugsweise Ethanol oder Isopropanol, sowie wahlweise der Zugabe von Detergenzien. So beschreiben EP-A-1502951, EP-A-1526176 und EP-A-1626085 (Agilent) Methanol, Ethanol, Isopropanol und Polyethyleneglycol als organischer Bindeverstärker in Kombination mit einer Chaotropsalzlösung, um Nukleinsäuren an einer festen Matrix zu binden. EP-A-1524317 (Roche) beschreibt die Verwendung von Aceton, Acetylaceton, Acetonitril, Dimethylsulfoxid, Diethylketon, Methylethylketon, Methylpropylketon, Isobutylmethylketone, γ-Butyrolacton, γ-Valerolacton, Propylencarbonat und N-Methyl-2-pyrrolidon zur Bindung von Nukleinsäuren an ein mineralisches Substrat. Dabei wird ein Gemisch bestehend aus einem wässrigen Puffer, Salze in hoher Konzentration und einen der vorstehend genannten organischen Komponenten eingesetzt. EP-A-2137309 (Stratagene) beschreibt die Verwendung von Sulfolan zur Isolierung von Nukleinsäuren durch das in Kontaktbringen einer Nukleinsäure-haltigen Probe in der Anwesenheit von Salzen und Sulfolan an ein mineralisches Substrat, welches mindestens eine Nukleinsäure absorbiert. EP-B-2322613 (Chomczynski) beschreibt eine Methode zur Isolierung von RNA aus einer RNA-haltigen Probe durch Behandlung der Probe mit einem monophasischen, phenolhaltigen Reagenz und Zugabe eines wasserlöslichen, organischen Lösemittels, um RNA zu präzipitieren. Das organische Lösemittel umfasst niedrigere Alkohole, Polyalkohole, Aceton, Ethylenglykoldiacetat und Methylsulfoxid. US20170121702 (Axxagarius) beschreibt die Verwendung einer RNA-Binding Solution zur Anbindung von RNA an einer festen Phase, bestehend aus mindestens einem chaotropen Agenz und einem organischen Lösemittel aus der Gruppe, Ethlyencarbonat, Ethlyenglykoldiacetat und 2-pyrrolidone oder deren Kombination. EP-A-1354036 (Life Technologies) beschreibt eine Verfahren zur Isolierung von Nukleinsäuren durch Aufschluss einer biologischen Probe mittels eines kationischen Tensids und einer Protease, wobei die Anbindung an eine feste Matrix durch Zugabe eines nicht-ionischen Tensids und einem Puffer mit hohen Salzkonzentrationen erfolgt. US2002192667 beschreibt Verfahren zur Isolierung mit Hilfe organischer Lösungsmittel und einer Festphase.

Alternative Verfahren sind zwar bekannt, beruhen jedoch meist immer noch auf der Zugabe von einer der obigen Substanzen, z. B. Isopropanol oder Chaotropsalz. Bisher sind nur wenige Verfahren bekannt die gänzlich auf die Verwendung von Chaotropsalz und Alkohol verzichten.

Aufgrund der bekannten Nachteile von Chaotropsalz/Alkohol-basierenden Reagenziensystemen wurde sich für die Bereitstellung von alternativen Reagenziensystemen auf sogenannten "green solvents" fokussiert. Bei den sogenannten "green solvents" handelt es sich um organische Lösungsmittel, die eine nicht-gefährliche/nicht-gesundheitsschädliche Alternative zu toxischen, reizenden oder gefährlichen Stoffen darstellen und aktuell mehr und mehr in verschiedenen Life Science Bereichen an Bedeutung gewinnen. Da die eingesetzten Komponenten bzw. deren Kombinationen bisher selten im Zusammenhang mit DNA-Isolierung genannt werden, soll eine Patentanmeldung des Verfahrens vorgenommen werden, um die Verwendung der speziellen Bindelösung im Kontext der DNA-Isolierung zu schützen.

### Kurze Beschreibung der Erfindung

Es wurde nun gefunden, dass die Verwendung einer Bindunglösung, bestehend aus mindestens einem zyklischen Alkylencarbonat oder einem Alkylenglykoldiacetat und einem nicht-ionischen Tensid, sich vorteilhaft auf die Anbindung von DNA an carboxylierte, magnetische Partikel auswirkt. Die gefundenen spezifischen Bindereagenziensysteme mit "green solvent" Komponenten sind für die Nukleinsäure-Isolation eines recht breites Probenspektrum ("Hard-to-Lyse") in Kombination mit verschiedenen Lyseverfahren (mechanische Lyse und enzymatische Lyse) geeignet, wobei nicht nur für den Bindeschritt sondern auch die übrigen Lyse, Wasch- und Isolationsschritte weitestgehend auf gefährliche oder reizende Komponenten wie Chaotropsalz oder Alkohol verzichtet werden kann, so dass diese kein Bestandteil des Verfahrens und des hierfür geeigneten Kits sind. Die Erfindung betrifft somit:
(1) Ein Verfahren zur Isolierung von Nukleinsäuren aus einer biologischen Probe, umfassend
   (a) Aufschluss der biologischen Probe mittels mechanischer und/oder enzymatischer Lyse und unter Verwendung einer chaotropsalzfreien Lyselösung,
   (b) Zugabe einer chaotropsalzfreien Bindelösung, umfassend wenigstens ein organisches Lösungsmittel, das aus zyklischen C₃₋₄ Alkylencarbonaten, C₂₋₃ Alkylenglykoldiacetaten und Derivaten derselben ausgewählt ist, und wenigstens ein nicht-ionisches Tensid, und carboxylierte, superparamagnetische Partikel mit einer Partikelgröße von 0,5 - 10 µm als Nukleinsäuren-bindende feste Phase zu der aufgeschlossenen Probe und Abtrennen der festen Phase mit den daran gebundenen Nukleinsäuren,
   (c) ein- oder mehrfaches Waschen der abgetrennten festen Phase mit den daran gebundenen Nukleinsäuren mit gleichen oder unterschiedlichen chaotropsalzfreien Waschlösungen, und
   (d) Desorption der Nukleinsäuren von der festen Phase durch Zugabe eines wässrigen chaotropsalzfreien Elutionspuffers;
(2) eine chaotropsalzfreie Bindelösung zur Isolierung von Nukleinsäuren aus einer biologischen Probe mittels carboxylierter, superparamagnetischer Partikel mit einer Partikelgröße von 0,5 - 10 µm gemäß dem Verfahren nach Aspekt (1), umfassend wenigstens ein organisches Lösungsmittel, das ausgewählt ist aus zyklischen C₃₋₄ Alkylencarbonaten, C₂₋₃ Alkylenglykoldiacetaten und Derivaten derselben, und wenigstens ein nicht-ionisches Tensid; und
(3) ein Kit zur Isolierung von Nukleinsäuren aus einer biologischen Probe mittels carboxylierter, superparamagnetischer Partikel mit einer Partikelgröße von 0,5 - 10 µm gemäß dem Verfahren nach Aspekt (1), wobei der Kit eine chaotropsalzfreie Bindelösung nach Aspekt (2), und optional carboxylierte, superparamagnetische Partikel mit einer Partikelgröße von 0,5 - 10 µm als Nukleinsäuren-bindende feste Phase umfasst.

### Kurzbeschreibung der Figuren

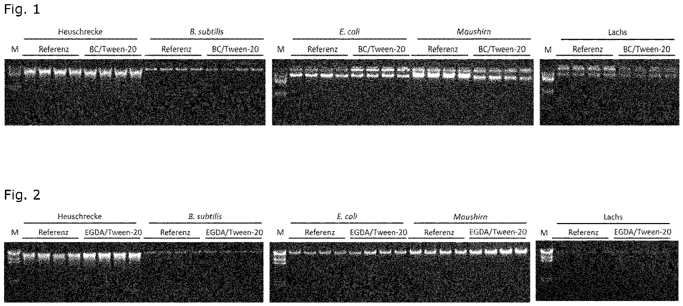

Figuren 1, 2 und 3 zeigen Ergebnisse der Beispiele 4 und 7.

### Detaillierte Beschreibung der Erfindung

Aspekt (1) der Erfindung betrifft ein Verfahren zur Nukleinsäure-Isolierung aus einer biologischen Probe. Dies umfasst die Isolation von DNA und RNA, sowie Gemischen und Derivaten derselben. Die Probe (z. B. Zellen, Gewebe) wird entweder mittels mechanischer oder enzymatischer Lyse unter Verwendung einer chaotropsalzfreien Lyselösung aufgeschlossen. Diese Lyselösungen enthalten vorzugsweise eine geringe Konzentration an EDTA (≤ 500 mM) und/oder an einem weiteren anionischen Tensid (≤ 2,5 % (w/v) SDS (Natriumdodecylsulfat). Nachfolgend wird die DNA durch Zugabe von magnetischen Partikeln und der chaotropsalzfreien Bindelösung an die feste Phase, in diesem Fall die magnetischen Partikel, gebunden. Die feste Phase wird durch in Kontaktbringen mit einer oder mehreren chaotropsalzfreien Waschlösungen gewaschen und anschließend von der festen Phase eluiert.

In dem Verfahren von Aspekt (1) der Erfindung erfolgt der Aufschluss mittels mechanischer und/oder enzymatischer Lyse und unter Verwendung einer chaotropsalzfreien Lyselösung, wobei die Lyselösung vorzugsweise geringe Konzentrationen an EDTA und/oder einem anionischen Tensid enthält.

In dem Verfahren von Aspekt (1) der Erfindung sind alle der genannten Lösungen und Puffer chaotropsalzfrei.

"Chaotropsalzfrei" im Sinne der vorliegenden Erfindung bedeutet, dass keine der dem Fachmann als chaotrop bekannten Verbindungen, wie Thiocyanate, Isothiocyanate, Perchlorate, Trichloracetate, Trifluoracetate, Iodide und/oder Guanidiniumsalze, wie insbesondere Guanidiniumhydrochlorid, Guanidiniumthiocyanat, Guanidiniumisothiocyanat, Natriumthiocyanat, Natriumiodid, Natriumperchlorat, Natriumtrichloracetat, Natriumtrifluoracetat, Harnstoff, Thioharnstoff und Phenol, die die Sekundärstuktur des Nukleinsäuren beeinflussen, in der entsprechenden Lösung oder dem entsprechenden Puffer vorhanden sind. Die "Lösungen" im Sinne der vorliegenden Erfindung müssen nicht notwendigerweise (nicht-chaotrope) anorganische oder organische Salze und Puffersubstanzen enthalten, sie können wie im Falle der Bindelösung oder einiger Waschlösungen vollständig frei davon sein. Die "Lösungen" und der "Elutionspuffer" im Sinne der vorliegenden Erfindung bestehen somit, soweit nicht anders angegeben aus wässrigen Lösungen bzw. wässrigen alkoholischen Lösungen (mit Ethanol oder Isopropanol als Alkohohlkomponenten, und einem Alkoholanteil von 35 bis 100 % (v/v), die mit geeigneten Substanzen (z. B. anorganische oder organische Salzen oder dem Fachmann bekannte Puffersubstanzen in ausreichender Menge, d.h. bis zu etwa 3 M) versetzt sind.

Es ist bevorzugt, dass in der Bindelösung das zyklisches C₂₋₄ Alkylencarbonat aus Butylencarbonat, Propylencarbonat, Ethylencarbonat, und Derivaten derselben ausgewählt ist, das C₂₋₃ Alkylenglykoldiacetat aus Ethylenglykoldiacetat, Propylenglycoldiacetat und Derivaten derselben ausgewählt ist. Derivate im Sinne der Vorliegenden Erfindung sind solche der genannten Verbindungen, in denen ein oder mehrere der Wasserstoffatome der Kohlenstoffkette(n) der Verbindungen unabhängig voneinander durch C₁₋₃ Alkyl-, C₁₋₃ Alkoxy-, Hydroxy- oder Halogensubstituenten ersetzt ist. Vorzugsweise enthält die Bindelösung wenigstens eine Verbindung, die ausgewählt ist aus Butylencarbonat (BC) (CAS 4437-85-8), Propylencarbonat (PC) (CAS 108-32-7), Ethlyenglykoldiacetat (EGDA) (CAS 111-55-7) und Propylenglykoldiacetat (PGDA) (CAS 623-84-7), wobei BC, PC und EGDA besonders bevorzugt sind.

Das wenigstens eine nicht-ionische Tensid ist vorzugsweise ausgewählt aus Polyoxyethylenderivaten von Sorbitanmonolaureat, Sorbitanmonopalmitat und Sorbitanmonooleat und Polyoxyethylenderivate, d.h. Verbindungen der Tween^{®} Serie, und Polyoxyethylenderivaten von Fettalkoholen, d.h. Verbindungen der Brij^{®} Serie, wobei Polyoxyethylen(20)-sorbitanmonolaureat (Tween^{®}-20; CAS 9005-64-5) besonders bevorzugt ist.

Dabei ist bevorzugt, dass das Verhältnis (v/v) von organischem Lösungsmittel (d.h. zyklischem C₂₋₄ Alkylencarbonat/C₂₋₃ Alkylenglykoldiacetat) zu nicht-ionischem Tensid, insbesondere dann, wenn das nicht-ionische Tensid Tween^{®}-20 ist, 80:20 bis 50:50, vorzugsweise 75:25 bis 55:45 und besonders bevorzugt etwa 70:30 beträgt. Die in Schritt (b) eingesetzte Nukleinsäuren-bindende feste Phase sind carboxylierte, superparamagnetische Partikel mit Partikeln im Größenbereich von 0,5 - 10 µm.

In Schritt (b) liegt die Menge an zugegebener chaotropsalzfreien Bindelösung, im Verhältnis zum Volumen der aufgeschlossenen Probe, bei 10:1 bis 1:10, vorzugsweise bei 3:1 bis 1:3 und besonders bevorzugt bei etwa 2:1 bis 1:1 (v/v) (Bindelösung/Lösung:aufgeschlossene Probe). Die Menge an zugegebener fester Phase im Verhältnis zum Volumen der aufgeschlossenen Probe beträgt 10:1 bis 50:1, vorzugsweise 20:1 bis 40:1 und besonders bevorzugt etwa 25:1 (v/v) (feste Phase: aufgeschlossene Probe).

Die chaotropsalzfreien Waschlösungen in Schritt (c) umfassen salzhaltige wässrige Lösungen, vorzugsweise salzhaltige und ethanolhaltige wässrige Lösungen, oder Ethanol-Wasser Gemische (z. B. 80 % Ethanol, 20 % Wasser; mit 0 bis 10 % Natriumacetat (w/v), 0 - 2 % Tween^{®} 20 (w/v), 0 - 9 % Essigsäure (v/v)). Das Volumen der jeweiligen Waschlösung beträgt das 10- bis 50 fache der festen Phase (carboxylierte, superparamagnetische Partikel mit Partikeln im Größenbereich von 0,5 - 10 µm).

Die chaotropsalzfreie Elutionslösung von Schritt (d) ist vorzugsweise Wasser oder ein alkalischer Niedrigsalzpuffer (zum Beispiel eine wässrige Lösung enthaltend 5 mM Tris/HCl, pH 8.5). Das Volumen des Elutionspuffers beträgt das 1- bis 20-fache der festen Phase.

Nachfolgend wird ein detaillierter Ablauf des erfindungsgemäßen Verfahrens skizziert.
1. Aufschluss einer biologischen Probe mittels mechanischer und/oder enzymatischer Lyse mittels Proteinase K und der Verwendung einer nicht-Chaotropsalz-enthaltenen Lyselösung (bestehend aus 0,4 bis 2,5 % SDS (w/v) und 10 - 400 mM EDTA).
2. Zugabe von 320 µl einer Bindelösung, bestehend aus einem zyklischem Alkylencarbonats oder Alkylenglykoldiactetats und eines Polysorbats im Verhältnis 70:30 (w/v) sowie carboxylierter, superparamagnetischer Partikel mit Partikeln im Größenbereich von 0,5 - 10 µm, zum Beispiel NucleoMag^{®} B-Beads des Kits NucleoMag^{®} Tissue (MACHEREY-NAGEL, REF: 744300.1), 24 µl als feste Phase zur Anbindung der Nukleinsäure.
3. Die magnetischen Partikel werden wahlweise mit einer oder mehreren salzhaltigen und ethanolhaltigen Waschlösungen oder Ethanol-Wasser Gemischen (z. B. 80 % Ethanol, 20 % Wasser (v/v) mit 0 bis 12,5 % Natriumacetat (w/v)) gewaschen. Alternativ können chaotropsalzhaltige und ethanolhaltige Waschlösungen, z. B. Lösung W1, W2, die einen hohen Chaotropsalzgehalt von 5 - 20 % (w/v) aufweisen, eingesetzt werden, was jedoch nicht Gegenstand der beanspruchten Erfindung ist.
4. Die magnetischen Partikel werden mittels einer alkoholischen Waschlösung (z. B. 80 % Ethanol, 20 % Wasser (v/v)) gewaschen.
5. Die Nukleinsäure wird mit Hilfe von Wasser oder eines leicht alkalischen Niedrigsalz-Puffer (z.B. 5 mM Tris/HCl, pH 8,5) von den magnetischen Partikeln gelöst.

Die Verwendung der zuvor beschriebenen chaotropsalzfreien Bindelösung ist im Vergleich zu anderen Lösung- oder Reagenzienkompositionen vorteilhaft, da diese:
- im Vergleich zu Ethanol oder Isopropanol schwer entzündlich sind;
- weniger toxisch oder gefährlich sind und somit umweltfreundlicher sind,
- den Einsatz von Chaotropsalz vermeiden; und
- abhängig vom Probenmaterial - gleiche oder bessere Ausbeuten und Reinheiten an isolierter DNA ermöglichen.

Der Kit von Aspekt (4) kann neben der chaotropsalzfreien Bindelösung und der Nukleinsäuren-bindende feste Phase weiterhin eine oder mehrere der chaotropsalzfreien Lyselösungen, der chaotropsalzfreien Waschlösungen und der chaotropsalzfreien Elutionspuffer wie vorstehend definiert enthalten.

Die Erfindung wird anhand der folgenden Beispiele näher erläutert.

Beispiele/Experimente, die Chaotropsalze verwenden, stellen Referenzbeispiele dar, die nicht Gegenstand der beanspruchten Erfindung sind. Diese sind, soweit möglich, explizit als solche gekennzeichnet.

### Beispiele

### Materialien und Methoden

Die folgenden Verbindungen und Zusammensetzungen wurden in den nachfolgenden Beispielen verwendet: Butylencarbonat (CAS 4437-85-8) (> 98,0 %, TCI Chemicals), Propylencarbonat (CAS 108-32-7) (99,7 %, Sigma-Aldrich), Ethlyencarbonat (CAS 96-49-1) (> 99,0 %, TCI Chemicals) Ethlyenglykoldiacetat (CAS 111-55-7) (99 %, Sigma-Aldrich), Propylenglykoldiacetat (CAS 623-84-7) (≥ 99,7 %, Sigma-Aldrich), Tween^{®} 20 (Sigma-Aldrich), 40 (Sigma-Aldrich), 60 (Sigma-Aldrich) und 80 (Sigma Aldrich), Brij^{®} O10 und 58 (Sigma-Aldrich), Natriumdodecylsulfat (SDS) (> 98,5 %, Sigma-Aldrich), Natrium-N-Lauroylsarcosin (≥ 94 %, Sigma-Aldrich), Triton X-100(Sigma-Aldrich), Span^{®} 40 (Sigma-Aldrich), 60 (Sigma-Aldrich), 65 (Sigma-Aldrich), 80 (Sigma-Aldrich), 83 (Sigma-Aldrich) und 85 (Sigma-Aldrich), Ethylendiamintetraessigsäure (EDTA, ≥ 99 %, Carl Roth GmbH), die Lösung A (Chaotropsalz- und Ethanolhaltiger-Bindelösung bestehend aus 50 % Ethanol (v/v) und 1,8 M Natriumperchlorat) und Lösung B (Chaotropsalz- und Ethanolhaltiger-Bindelösung bestehend aus 2,4 M Natriumperchlorat und 60 % Ethanol (v/v)); die Lösungen W1 und W2, bestehend aus 1,3 M Natriumperchlorat und 35 % Ethanol (v/v), die einen hohen Gehalt an Chaotropsalz (5 - 20 % (w/v)) und Ethanol (20 - 35 % (v/v)) aufweisen, die Lösung W3 bestehend aus 1,5 M Natriumacetat und 50 % Ethanol (v/v), die kein Chaotropsalz enthält.

### Beschreibung des Standardprotokolls

Die beispielhaft aufgeführten Experimente wurden, sofern nicht anders erwähnt, nachfolgenden Protokoll durchgeführt.

Enzymatischer Probenaufschluss: Je Extraktion wurde eine spezifizierte Menge Probe mit einer Lyselösung (je 200 µl) und Proteinase K (je 25 µl einer 30 mg/ml-Lösung) schüttelnd bei 56 °C für 1-16 h in einem geeigneten Reaktionscontainer inkubiert, um einen Aufschluss der biologischen Probe zu erreichen (nachfolgend "Lysat" genannt). Eine optionale, anschließende Zentrifugation des Lysats, z. B. für 5 min bei 5,600 - 6000 x g, pelletiert unverdaute Schwebstoffe.

Versetzen mit Bindelösung: Dem geklärten Lysat wurden je Extraktion 320 µl der jeweiligen Bindelösung und 24 µl einer magnetischen Beadsuspension (carboxylierte, superparamagnetische Partikel enthaltene Partikel mit einem Größenbereich von 0,5 - 10 µm, zum Beispiel NucleoMag^{®} B-Beads des Kits NucleoMag^{®} Tissue (MACHEREY-NAGEL, REF: 744300.1)) hinzugefügt und die Lysat-Bead-Bindelösungs-Mischung mittels Auf- und Ab-pipettieren durchmischt. Wahlweise können für die Durchmischung auch andere Techniken, wie die Verwendung eines Laborschüttlers oder eines automatisierten Magnetpartikelprozessierungsgeräts, verwendet werden. Abtrennen und Waschen: Nach erfolgter Durchmischung werden die magnetischen Partikel mittels eines statischen oder automatisierten Magnetseparators abgetrennt, so dass entweder der Lysat-Bindelösungs-enthaltene Überstand mittels Aspiration oder die magnetischen Partikel mittels eines beweglichen Magnetseparators separiert werden können. Die magnetischen Partikel werden darauffolgend in 800 µl der jeweiligen Waschlösung, zum Beispiel durch Auf- und Ab-pipettieren, Schütteln oder andere Techniken resuspendiert bzw. durchmischt. Nach vollständiger Durchmischung erfolgt eine erneute Abtrennung der magnetischen Partikel von dem jeweiligen Waschpuffer durch eine der oben beschriebenen Techniken. Im weiteren Verlauf erfolgen zwei weitere Waschschritte, mit den jeweiligen Waschlösungen (je 800 µl) nach dem zuvor beschriebenen Prinzip, wobei im dritten Waschschritt ein 80 %iges Ethanol-Wasser-Gemisch als Waschlösung verwendet wird.

Elution: Nach einem Trocknungsschritt für 5 - 10 min bei Raumtemperatur, wurde die DNA durch Zugabe von 100 µl Elutionpuffers (5 mM Tris/HCl, pH 8,5) von den magnetischen Partikeln gelöst und mit Hilfe eines Magnetseperators, wie zuvor beschrieben, abgetrennt.

### Beispiel 1 (Referenzbeispiel)

Je Extraktion wurden 20 mg eines tierischen Gewebes (Rehleber für #1-17; Schweineniere #18-19) mittels einer SDS-haltigen Lyselösung (200 µl je Probe; 1 % SDS (w/v), 100 mM EDTA, 10 mM Tris) und Verwendung von Proteinase K (ca. 30 mg/ml) schüttelnd bei 56 °C über Nacht lysiert. Anschließend wurden je Probe 320 µl der jeweiligen Bindelösung und 24 µl (carboxylierte, superparamagnetische Partikel mit Partikeln im Größenbereich von 0,5 - 10 µm) NucleoMag^{®} B-Beads hinzugegeben. Die nachfolgende Tabelle 1 zeigt relative und absolute Ausbeute an DNA in Abhängigkeit der unterschiedlichen Bindelösungszusammensetzungen (v/v) im Vergleich zu Referenzpuffern.

**Tabelle 1**

| # | Substanz A | Substanz B | DNA Ausbeute [µg] | Relative Ausbeute [%] |
|---|---|---|---|---|
| 1 | BC 100 % | - | 1,4 | 4,50 |
| 2 | BC 80 % | Tween^{®}20 20 % | 2,0 | 6,43 |
| 3 | BC 70 % | Tween^{®}20 30 % | 14,3 | 45,98 |
| 4 | BC 60 % | Tween^{®}-20 40 % | 26,2 | 84,24 |
| 5 | PC 100 % | - | 1,3 | 4,18 |
| 6 | PC 80 % | Tween^{®}20 20 % | 2,6 | 8,36 |
| 7 | PC 70 % | Tween^{®}20 30 % | 28,8 | 92,60 |
| 8 | PC 60 % | Tween^{®}20 40 % | 28,1 | 90,35 |
| 9 | EC 100 % | - | nicht messbar | - |
| 10 | EC 80 % | Tween^{®}20 20 % | nicht messbar | - |
| 11 | EC 70 % | Tween^{®}20 30 % | 34,0 | 109,32 |
| 12 | EC 60 % | Tween^{®}20 40 % | 35,2 | 113,18 |
| 13 | EGDA 100 % | - | 1,5 | 4,82 |
| 14 | EGDA 80 % | Tween^{®}20 20 % | 26,8 | 86,17 |
| 15 | EGDA 70 % | Tween^{®}20 30 % | 29,4 | 94,53 |
| 16 | EGDA 60 % | Tween^{®}20 40 % | 31,8 | 102,25 |
| 17 | Lösung B (Referenz) | | 31,1 | 100 |
| 18 | - | Tween^{®}20 35 % | 0,4 | 6,78 |
| 19 | Lösung B (Referenz) | | 5,9 | 100 |
| 20 | PGDA 100 % | - | 0,7 | 3,77 |
| 21 | PGDA 80 % | Tween^{®}20 20 % | 1,3 | 6,76 |
| 22 | PGDA 70 % | Tween^{®}20 30 % | 16,0 | 84,89 |
| 23 | PGDA 60 % | Tween^{®} 20 40 % | 14,4 | 76,46 |
| 24 | Lösung A (Referenz) | | 18,9 | 100 |

Die Lysat-Bead-Bindelösungs-Mischung wurde anschließend gemäß den Angaben des vorstehend beschriebenen Standardprotokolls automatisiert prozessiert, wobei die magnetischen Partikel zweimal mit je 800 µl der Waschlösung W1 und mit je 800 µl 80 % Ethanols gewaschen wurden. Nach einem Trocknungsschritt wurde die DNA durch Zugabe von 100 µl Elutionspuffers (5 mM Tris/HCl, pH 8,5) von den magnetischen Partikeln gelöst und abgetrennt. Die DNA-Ausbeute wurde mittels UV-Spektrometrie bestimmt und in Relation zur Referenzlösung Lösung B (#17/ #19) oder Lösung A (#24) gesetzt (Chaotropsalz- und Ethanolhaltige-Bindelösung). Anhand der Daten ist ersichtlich, dass Mischungen von BC, PC, EC oder EGDA und Tween^{®}20 als alternative Bindelösung für Chaotropsalz- und alkoholhaltigen Bindelösung, während der DNA-Isolierung, eingesetzt werden kann.

### Beispiel 2

Die DNA-Isolierung erfolgte aus mechanisch aufgeschlossenen, fetthaltigen Probenmaterial. Das Probenmaterial wurde mit 140 µl einer EDTA-haltigen wässrigen Lösung (500 mM EDTA) mittels mechanischer Lyse unter Verwendung von 5 x 3 mm Stahlkugeln in einem 2 ml Schraubdeckelgefäß für 1 min bei 10 Hz gefolgt von 1 min bei 20 Hz in einer Schwingmühle aufgeschlossen. Zusätzlich wurde hier die Waschlösung W2 durch die Waschlösung W3, bestehend aus 50 % Ethanol und 50 % einer 3 M Natriumacetat-Lösung, ausgetauscht, um die Verwendung von Chaotropsalz in den Waschlösungen zu vermeiden. Die nachfolgende Tabelle 2 zeigt relative und absolute Ausbeute an DNA in Abhängigkeit der unterschiedlichen Zusammensetzungen der Bindelösungen, die im Verhältnis 70:30 (v/v; siehe unten) verwendet wurden. Als Referenz wurde ein auf Chaotropsalz- und Ethanolbasierendes Kit verwendet.

**Tabelle 2**

| Extraktion | BC/ Tween^{®}20 | PC/ Tween^{®}20 | EC/ Tween^{®}20* | EGDA/ Tween^{®}20 | Referenz: NucleoSpin^{®} Lipid Tissue* |
|---|---|---|---|---|---|
| 1 | 3,69 | 2,5 | 0,38 | 3,96 | 3,4 |
| 2 | 2,22 | 3,81 | 0,35 | 2,63 | 3,9 |
| 3 | 4,31 | 2,13 | 0,36 | 2,42 | 4,1 |
| 4 | 4,03 | 2,79 | 0,42 | 3,5 | 6,8 |
| Mittelwert | 3,6 | 2,8 | 0,4 | 3,1 | 4,6 |

| | | | | | |
|---|---|---|---|---|---|
| * Referenzbeispiel | | | | | |

Anhand der Daten ist ersichtlich, dass Mischungen eines zyklischen Alkylencarbonats oder Ethylenglykoldiacetat und eines Polysorbats als alternative Bindelösung für Chaotropsalz- und alkoholhaltige Bindelösungen, während der DNA-Isolierung, eingesetzt werden kann.

### Beispiel 3 (Referenzbeispiel)

Je Extraktion wurden 20 mg eines tierischen Gewebes (Rehleber für #1-17; Schweineniere #18-19) mittels einer SDS- und, EDTA-haltigen Lyselösung (200 µl je Probe; 1 % SDS (w/v), 100 mM EDTA, 10 mM Tris) und Verwendung von Proteinase K (30 mg/ml) gemäß den Angaben des vorstehend beschriebenen Standardprotokolls prozessiert. Anschließend wurden je Probe 320 µl der jeweiligen Bindelösung und 24 µl NucleoMag^{®} B-Beads (carboxylierte, superparamagnetische Partikel mit Partikeln im Größenbereich von 0,5 - 10 µm) hinzugegeben. Die nachfolgenden Bindelösungszusammensetzungen wurden im Verhältnis 70:30 (v/v) verwendet. Die weitere Prozessierung erfolgte gemäß den unter Beispiel 1 aufgeführten Angaben. Die DNA-Ausbeute wurde mittels UV-Spektrometrie bestimmt und in Relation zur Referenzlösung Lösung A (#7) gesetzt. Die nachfolgende Tabelle 3 zeigt relative und absolute Ausbeute an DNA in Abhängigkeit der unterschiedlichen Bindelösungzusammensetzungen.

**Tabelle 3**

| # | Substanz A | Substanz B | DNA Ausbeute [µg] | Relative Ausbeute [%] |
|---|---|---|---|---|
| 1 | BC | Tween^{®}20 | 24,6 | 113,16 |
| 2 | BC | Tween^{®}40 | 20,4 | 94,12 |
| 3 | BC | N-Lauroylsarcosin | 1,4 | 6,2 |
| 4 | BC | Triton X-100 | 1,5 | 6,92 |
| 5 | BC | SDS (10 %ige Stammlösung) | 1,0 | 4,39 |
| 6 | BC | Brij^{®} 58 | 0,7 | 3,31 |
| 7 | Lösung A (Referenz) | | 21,7 | |
| 8 | BC | Tween^{®}80 | 32,4 | 134,89 % |
| 9 | BC | Span^{®} 40 | 2,8 | 11,61 |
| 10 | BC | Span^{®} 60 | 1,9 | 7,98 |
| 11 | BC | Span^{®} 65 | 2,6 | 10,81 |
| 12 | BC | Span^{®} 80 | 9,7 | 40,13 |
| 13 | BC | Span^{®} 83 | 8,6 | 35,72 |
| 14 | BC | Span^{®} 85 | 6,6 | 27,42 |
| 15 | BC | Brij^{®} O10 | 13,9 | 57,80 |
| 16 | Lösung A (Referenz) | | 24,1 | |

Anhand der Daten ist ersichtlich, dass Mischungen von BC und eines Polysorbats als alternative Bindelösung für Chaotropsalz- und alkoholhaltige Bindelösungen, während der DNA-Isolierung, eingesetzt werden kann und vorteilhaft ist.

### Beispiel 4 (Referenzbeispiel)

Um die Vielseitigkeit der Methode zu demonstrieren, wurde aus schwierigen, sogenannten "Hard-to-Lyse" Probenmaterialien unterschiedlicher Organismen DNA isoliert. Dazu zählen neben Insekten und Mikroorganismen auch besonders fetthaltige Gewebe wie Hirn- oder Fischproben. Ein bekanntes Problem ist hier, die Trübung der Eluate durch eine nicht-ausreichende Entfernung von Kontaminationen. Die nachfolgenden Probenmaterialien und Mengen wurden verwendet:
Heuschrecke 40 mg
*Bacillus subtilis* ca. 1 x 10⁹ Zellen
*Escherichia coli* ca. 1 x 10⁹ Zellen
Maushirn 20 mg
Lachs 20 mg

Das entsprechende Probenmaterial wurde mit Hilfe von 200 µl einer SDS- und EDTAhaltigem Lyselösung (1 % SDS (w/v), 100 mM EDTA, 10 mM Tris) und 25 µl Proteinase K (ca. 30 mg/ml) entsprechend den Angaben der in Beispiel 1 beschriebenen Prozedur schüttelnd über Nacht bei 56 °C lysiert. Die Lysate wurden anschließend nach Zugabe von 20 µl RNase A (20 mg/ml) für 5 min bei Raumtemperatur inkubiert und mittels Zentrifugation (4500 x g, 20 min) geklärt. Je Probe wurden 225 µl des geklärten Lysats überführt und gemäß den Angaben der zuvor in Beispiel 1 beschriebenen Prozedur prozessiert. Als Bindelösung wurde eine Mischung von BC/Tween^{®}20 und EGDA/Tween^{®}20 im Verhältnis 70 zu 30 (v/v) verwendet. Die Bindelösung Lösung A wurde als Referenz verwendet.

Die Quantität und Qualität der isolierten DNA wurden mittels Agarose-Gel-Elektrophorese und einer fluormetrischen Quantifizierungsmethode analysiert. Die nachfolgende Tabelle 4 zeigt absolute DNA-Ausbeuten (Mittelwert) im Vergleich zum Mittelwert des entsprechenden Referenzbeispiels (Puffer A).

**Tabelle 4**

| Probenmaterial | Bindelösung | DNA Ausbeute [µg] | A260/ A280 | A260/ A230 | Trübung der Eluate |
|---|---|---|---|---|---|
| Heuschrecke | BC/Tween^{®}20 | 11,9 | 1,7 | 1,9 | - |
| | Lösung A | 15,5 | 1,4 | 1,1 | trüb |
| *Bacillus subtilis* | BC/Tween^{®}20 | 3,7 | 1,5 | 1,0 | - |
| | Lösung A | 1,3 | 1,5 | 1,1 | - |
| *Escherichia coli* | BC/Tween^{®}20 | 4,0 | 1,7 | 1,3 | - |
| | Lösung A | 3,4 | 1,5 | 0,8 | - |
| Maushirn | BC/Tween^{®}20 | 3,7 | 1,7 | 1,4 | - |
| | Lösung A | 8,2 | 1,3 | 0,9 | trüb |
| Lachs | BC/Tween^{®}20 | 0,7 | 1,5 | 0,8 | - |
| | Lösung A | 1,7 | 1,3 | 0,7 | trüb |

In Figur 1 sind TAE-Gele der isolierten DNA gezeigt. Je Probe wurden 3 µl des Eluats auf ein 1 % TAE-Gel aufgetragen (M: Lambda Hind III Marker).

Die nachfolgende Tabelle 5 zeigt absolute DNA-Ausbeuten (Mittelwert) im Vergleich zum Mittelwert des entsprechenden Referenzbeispiels (Puffer A).

**Tabelle 5**

| Probenmaterial | Bindelösung | DNA Ausbeute [µg] | A260/ A280 | A260/ A230 | Trübung der Eluate |
|---|---|---|---|---|---|
| Heuschrecke | EGDA/Tween^{®}20 | 29,5 | 1,7 | 1,9 | - |
| | Lösung A | 24,6 | 1,4 | 1,1 | trüb |
| *Bacillus subtilis* | EGDA/Tween^{®}20 | 2,6 | 1,6 | 1,1 | - |
| | Lösung A | 2,4 | 1,6 | 1,2 | - |
| *Escherichia coli* | EGDA/Tween^{®}20 | 9,2 | 1,5 | 0,9 | - |
| | Lösung A | 10,1 | 1,5 | 0,9 | - |
| Maushirn | EGDA/Tween^{®}20 | 17,2 | 1,7 | 1,5 | - |
| | Lösung A | 10,9 | 1,3 | 0,9 | trüb |
| Lachs | EGDA/Tween^{®}20 | 2,2 | 1,6 | 1,1 | - |
| | Lösung A | 2,9 | 1,3 | 0,7 | trüb |

In Figur 2 sind TAE-Gele der isolierten DNA gezeigt. Je Probe wurden 1 µl des Eluats auf ein 1 % TAE-Gel aufgetragen (M: Lambda Hind III Marker).

Anhand der hier dargestellten Daten ist erkennbar, dass sich eine Bindelösung, bestehend aus BC oder EGDA und eines Polysorbats (Tween^{®}20), als vorteilhaft hinsichtlich Reinheit und Ausbeute der isolierten DNA erweist.

### Beispiel 5 (Referenzbeispiel)

Je Probe wurden 20 mg Rehleber mit einer SDS-haltigem Lyselösung (1 % SDS (w/v), 100 mM EDTA, 10 mM Tris) und 25 µl Proteinase K (ca. 30 mg/ml) gemäß den Angaben des vorstehend beschriebenen Standardprotokolls lysiert. Anschließend wurden 320 µl der jeweiligen Bindelösung und 30 µl der jeweiligen magnetischen Partikel, silanisierte (NucleoMag^{®} P-Beads) oder carboxylierte (NucleoMag^{®} B-Beads) (superparamagnetische Partikel mit Partikeln im Größenbereich von 0,5 - 10 µm) hinzugefügt und schüttelnd inkubiert. Die magnetischen Partikel wurden anschließend gemäß den Angaben in Beispiel 1 prozessiert. Die nachfolgende Tabelle 6 zeigt absolute Ausbeute DNA bei Verwendung verschiedener Bindelösungen in Abhängigkeit der festen Phase.

**Tabelle 6**

| # | Bindelösung | | | |
|---|---|---|---|---|
| | Substanz A | Substanz B | Feste Phase | DNA Ausbeute [µg] |
| 1 | Butylencarbonat 65 % | Tween^{®}20 35 % | NucleoMag^{®} P-Beads | 0,6 |
| 2 | | | NucleoMag^{®} B-Beads | 7,4 |
| 3 | Propylencarbonat 65 % | Tween^{®}20 35 % | NucleoMag^{®} P-Beads | 1,0 |
| 4 | | | NucleoMag^{®} B-Beads | 9,7 |
| 5 | Ethylencarbonat 65 % | Tween^{®}20 35 % | NucleoMag^{®} P-Beads | 4,9 |
| 6 | | | NucleoMag^{®} B-Beads | 11,6 |
| 7 | Ethylenglykoldiacetat 65 % | Tween^{®}20 35 % | NucleoMag^{®} P-Beads | 0,5 |
| 8 | | | NucleoMag^{®} B-Beads | 11,2 |
| 9 | Lösung B (Referenz) | | NucleoMag^{®} P-Beads | 0,6 |
| 10 | | | NucleoMag^{®} B-Beads | 5,9 |

Die Daten zeigen, dass die Effizienz der Anbindung stark von der Art der Oberfläche der festen Phase abhängt. Zusätzlich ist erkenntlich, dass eine höhere Ausbeute an DNA mit Hilfe der hier aufgeführten Bindelösungskompositionen im Vergleich zum Puffer des Referenzbeispiels erzielt werden kann.

### Beispiel 6

Je Extraktion wurden 30 mg eines tierischen Gewebes (Schweineniere) mittels einer SDS- und EDTA-haltigen Lyselösung (je Extraktion 200 µl; 1 % SDS (w/v), 100 mM EDTA, 10 mM Tris) und Verwendung von Proteinase K (Je Extraktion 25 µl einer 30 mg/ml Lösung) gemäß den Angaben des vorstehend beschriebenen Standardprotokolls über Nacht lysiert. Anschließend wurden je Probe 320 µl einer EGDA/Tween^{®} 20 Bindelösung im Verhältnis 70:30 (v/v) und 24 µl NucleoMag^{®} B-Beads (carboxylierte, superparamagnetische Partikel mit Partikeln im Größenbereich von 0,5 - 10 µm) hinzugegeben. Als Waschlösung wurden entweder nicht-chaotropsalzhaltige Waschlösungskombination (W3/ W3/ 80 % EtOH) und chaotropsalz-haltige Waschlösungskombinationen (W2/ W2/ 80 %EtOH oder W1/ W2/ 80 % EtOH) eingesetzt. Die weitere Prozessierung erfolgte gemäß den unter Beispiel 1 aufgeführten Angaben.

Die DNA-Ausbeute und Reinheit wurde mittels UV-Spektrometrie bestimmt und in Relation zur Referenzlösung Lösung A (#7) gesetzt. Die nachfolgende Tabelle 7 zeigt absolute DNA-Ausbeuten (Mittelwert) im Vergleich zum Mittelwert der Referenz (Puffer A)

**Tabelle 7**

| Bindelösung | WaschlösungsKombinationen | DNA Ausbeute [µg] | A260/ A280 | A260/ A230 | Trübung der Eluate |
|---|---|---|---|---|---|
| EGDA/ Tween^{®}-20 | W3/ W3/ 80 % EtOH | 23,8 | 1,9 | 2,3 | - |
| | W2/ W2/ 80 % EtOH* | 22,4 | 1,9 | 2,3 | - |
| Lösung A | W1/ W2/ 80 % EtOH* | 19,8 | 1,8 | 2,2 | - |

| | | | | | |
|---|---|---|---|---|---|
| * Referenzbeispiel | | | | | |

Anhand der hier dargestellten Daten ist erkennbar, dass die zuvor beschriebene Bindelösung, ebenfalls mit nicht-chaotropsalzhaltigen Waschlösungen verwendet werden kann und sich als vorteilhaft hinsichtlich Reinheit und Ausbeute der isolierten DNA erweist.

### Beispiel 7 (Referenzbeispiel)

Je Extraktion wurden 20 mg eines tierischen Gewebes (Schweineniere) mittels einer SDS-und EDTA-haltigen Lyselösung (Je Extraktion 200 µl; 1 % SDS (w/v), 100 mM EDTA, 10 mM Tris) und Verwendung von Proteinase K (Je Extraktion 25 µl einer 30 mg/ml Lösung) gemäß den Angaben des vorstehend beschriebenen Standardprotokolls über Nacht lysiert. Anschließend wurden je Probe 24 µl NucleoMag^{®} B-Beads (carboxylierte, superparamagnetische Partikel mit Partikeln im Größenbereich von 0,5 - 10 µm) und 320 µl einer EGDA/Tween^{®}20 Bindelösung im Verhältnis 70:30 (v/v) oder eines Bindelösungsgemischs, bestehend aus EGDA und einer 1,8 M Chaotropsalzlösung (Guanidiniumhydrochlorid (GuHCl); Guanidiniumthiocyanat (GITC); Natriumperchlorat (NaClO4)) hinzugegeben. Die weitere Prozessierung erfolgte gemäß den unter Beispiel 1 aufgeführten Angaben.

Die DNA-Ausbeute und Reinheit wurde mittels UV-Spektrometrie bestimmt und in Relation zur Referenzlösung Lösung A oder der Bindelösung EGDA/Tween^{®}20 (70/30 (v/v)) gesetzt.

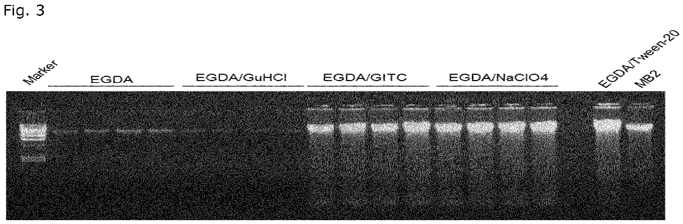

Figur 3 zeigt TAE-Gele der isolierten DNA. Je Probe wurden 1 µl des Eluats auf ein 1 % TAE-Gel aufgetragen. (M: Lambda Hind III Marker).

Die obigen Daten zeigen, dass die hier beschriebene Bindelösung gleichwertig zu einem chaotropsalzhaltigen Bindelösungsgemisch ist.

## Patentansprüche

1. Verfahren zur Isolierung von Nukleinsäuren aus einer biologischen Probe, umfassend
(a) Aufschluss der biologischen Probe mittels mechanischer und/oder enzymatischer Lyse und unter Verwendung einer chaotropsalzfreien Lyselösung,
(b) Zugabe einer chaotropsalzfreien Bindelösung, umfassend wenigstens ein organisches Lösungsmittel, das ausgewählt ist aus zyklischen C₃₋₄ Alkylencarbonaten, C₂₋₃ Alkylenglykoldiacetaten und Derivaten derselben, und wenigstens ein nicht-ionisches Tensid, und carboxylierte, superparamagnetische Partikel mit einer Partikelgröße von 0,5 - 10 µm als Nukleinsäuren-bindende feste Phase zu der aufgeschlossenen Probe und Abtrennen der festen Phase mit den daran gebundenen Nukleinsäuren,
(c) ein- oder mehrfaches Waschen der abgetrennten festen Phase mit den daran gebundenen Nukleinsäuren mit gleichen oder unterschiedlichen chaotropsalzfreien Waschlösungen, und
(d) Desorption der Nukleinsäuren von der festen Phase durch Zugabe eines wässrigen chaotropsalzfreien Elutionspuffers.

2. Verfahren nach Anspruch 1, wobei die chaotropsalzfreie Lyselösung geringe Konzentrationen an EDTA und/oder einem anionischen Tensid enthält.

3. Verfahren nach Anspruch 1 oder 2, wobei in der chaotropsalzfreien Bindelösung das zyklische C₃₋₄ Alkylencarbonat aus Butylencarbonat, Propylencarbonat und Derivaten derselben ausgewählt ist, das C₂₋₃ Alkylenglykoldiacetat aus Ethylenglykoldiacetat, Propylenglycoldiacetat und Derivaten derselben ausgewählt ist, und vorzugsweise die Bindelösung wenigstens eine Verbindung enthält, die ausgewählt ist aus Butylencarbonat, Propylencarbonat und Ethylenglykoldiacetat.

4. Verfahren nach einem oder mehreren der Ansprüche 1 bis 3, wobei in der chaotropsalzfreien Bindelösung
(i) das wenigstens eine nicht-ionische Tensid ausgewählt ist aus Polyoxyethylenderivaten von Sorbitanmonolaureat, Sorbitanmonopalmitat und Sorbitanmonooleat und Polyoxyethylenderivaten von Fettalkoholen, wobei Polyoxyethylen(20)-sorbitanmonolaureat besonders bevorzugt ist; und/oder
(ii) das Verhältnis (v/v) von organischem Lösungsmittel zu nicht-ionischem Tensid von 80:20 bis 50:50, vorzugsweise 75:25 bis 55:45 und besonders bevorzugt etwa 70:30 beträgt.

5. Verfahren nach einem oder mehreren der Ansprüche 1 bis 4, wobei in Schritt (b)
(i) die Menge an zugegebener chaotropsalzfreier Bindelösung im Verhältnis zum Volumen der aufgeschlossenen Probe von 10:1 bis 1:10, vorzugsweise 3:1 bis 1:3 und besonders bevorzugt etwa 2:1 bis 1:1 (v/v) beträgt; und/oder
(ii) die Menge an zugegebener fester Phase im Verhältnis zum Volumen der aufgeschlossenen Probe von 10:1 bis 50:1, vorzugsweise 20:1 bis 40:1 und besonders bevorzugt etwa 25:1 (v/v) beträgt.

6. Verfahren nach einem oder mehreren der Ansprüche 1 bis 5, wobei die chaotropsalzfreien Waschlösungen in Schritt (c), salzhaltige- und/oder Ethanolhaltige wässrige Lösungen oder salzhaltige ethanolische Lösungen, vorzugsweise chaotropsalzfreie wässrige Lösungen, chaotropsalzfreie Ethanol-Wasser Gemische oder chaotropsalzfreie ethanolische Lösungen sind.

7. Verfahren nach einem oder mehreren der Ansprüche 1 bis 6, wobei der chaotropsalzfreie Elutionspuffer von Schritt (d) ein alkalischer Niedrigsalzpuffer ist.

8. Chaotropsalzfreie Bindelösung zur Isolierung von Nukleinsäuren aus einer biologischen Probe mittels carboxylierter, superparamagnetischer Partikel mit einer Partikelgröße von 0,5 - 10 µm gemäß dem Verfahren nach einem oder mehreren der Ansprüche 1 bis 7, wobei die Bindelösung wenigstens ein organisches Lösungsmittel, das ausgewählt ist aus zyklischen C₃₋₄ Alkylencarbonaten, C₂₋₃ Alkylenglykoldiacetaten und Derivaten derselben, und wenigstens ein nicht-ionisches Tensid umfasst.

9. Chaotropsalzfreie Bindelösung nach Anspruch 8, die eine wie in Anspruch 4 definierte chaotropsalzfreie Bindelösung ist.

10. Chaotropsalzfreie Bindelösung nach Anspruch 8 oder 9, die weiterhin carboxylierte, superparamagnetische Partikel mit einer Partikelgröße von 0,5 - 10 µm als Nukleinsäuren-bindende feste Phase umfasst.

11. Kit zur Isolierung von Nukleinsäuren aus einer biologischen Probe mittels carboxylierter, superparamagnetischer Partikel mit einer Partikelgröße von 0,5 - 10 µm gemäß dem Verfahren nach einem oder mehreren der Ansprüche 1 bis 7, wobei der Kit eine chaotropsalzfreie Bindelösung nach einem oder mehreren der Ansprüche 8 bis 10 umfasst.

12. Kit nach Anspruch 11, weiterhin umfassend einen oder mehrere der chaotropsalzfreien Lyselösungen, der chaotropsalzfreien Waschlösungen und des chaotropsalzfreien Elutionspuffers, wie in den Ansprüchen 1 bis 7 definiert.

## Claims

1. A process for isolating nucleic acids from a biological sample, comprising:
(a) lysing the biological sample by mechanical and/or enzymatic lysis using a lysing solution that is free of chaotropic salts,
(b) adding to the lysed sample a binding solution free of chaotropic salts and comprising at least one organic solvent selected from cyclic C₃₋₄ alkylene carbonates, C₂₋₃ alkylene glycol diacetates, and derivatives thereof, at least one non-ionic surfactant, and carboxylated superparamagnetic particles with a particle size of from 0.5 to 10 µm as a nucleic acid-binding solid phase, and separating the solid phase with the nucleic acids bound thereto,
(c) washing the separated solid phase including the nucleic acids bound thereto once or several times with identical or different washing solutions that are free of chaotropic salts, and
(d) desorbing the nucleic acids from the solid phase by adding an aqueous elution buffer that is free of chaotropic salts.

2. The process according to claim 1, wherein said lysing solution free of chaotropic salts contains low concentrations of EDTA and/or an anionic surfactant.

3. The process according to claim 1 or 2, wherein, in the binding solution free of chaotropic salts, the cyclic C₃₋₄ alkylene carbonate is selected from butylene carbonate, propylene carbonate, and derivatives thereof, and the C₂₋₃ alkylene glycol diacetate is selected from ethylene glycol diacetate, propylene glycol diacetate, and derivatives thereof, and preferably the binding solution contains at least one compound selected from butylene carbonate, propylene carbonate, and ethylene glycol diacetate.

4. The process according to one or more of claims 1 to 3, wherein, in the binding solution free of chaotropic salts:
(i) said at least one non-ionic surfactant is selected from polyoxyethylene derivatives of sorbitan monolaureate, sorbitan monopalmitate and sorbitan monooleate, and polyoxyethylene derivatives of fatty alcohols, wherein polyoxyethylene(20) sorbitan monolaureate is more preferred; and/or
(ii) the ratio (v/v) of organic solvent to non-ionic surfactant is from 80:20 to 50:50, preferably from 75:25 to 55:45, and more preferably is about 70:30.

5. The process according to one or more of claims 1 to 4, wherein, in step (b),
(i) the amount of binding solution free of chaotropic salts that has been added as a ratio to the volume of lysed sample is from 10:1 to 1:10, preferably from 3:1 to 1:3, and more preferably from about 2:1 to 1:1 (v/v); and/or
(ii) the amount of solid phase added as a ratio to the volume of lysed sample is from 10:1 to 50:1, preferably from 20:1 to 40:1, and more preferably is about 25:1 (v/v).

6. The process according to one or more of claims 1 to 5, wherein the washing solutions free of chaotropic salts in step (c) are salt-containing and/or ethanol-containing aqueous solutions, or salt-containing ethanolic solutions, preferably non-chaotropic aqueous solutions, non-chaotropic ethanol-water mixtures, or non-chaotropic ethanolic solutions.

7. The process according to one or more of claims 1 to 6, wherein the non-chaotropic eluting buffer of step (d) is an alkaline low salt buffer.

8. A non-chaotropic binding solution for isolating nucleic acids from a biological sample by means of carboxylated superparamagnetic particles having a particle size of from 0.5 to 10 µm obtained by the process according to one or more of claims 1 to 7, wherein said binding solution comprises at least one organic solvent selected from cyclic C₃₋₄ alkylene carbonates, C₂₋₃ alkylene glycol diacetates, and derivatives thereof, and at least one non-ionic surfactant.

9. The non-chaotropic binding solution according to claim 8, which is a non-chaotropic binding solution as defined in claim 4.

10. The non-chaotropic binding solution according to claim 8 or 9, further comprising carboxylated superparamagnetic particles having a particle size of from 0.5 to 10 µm as a nucleic acid-binding solid phase.

11. A kit for isolating nucleic acids from a biological sample by means of carboxylated superparamagnetic particles having a particle size of from 0.5 to 10 µm obtained by the process according to one or more of claims 1 to 7, wherein said kit comprises a binding solution that is free of chaotropic salts according to one or more of claims 8 to 10.

12. The kit according to claim 11, further comprising one or more of the lysing solutions being free of chaotropic salts, washing solutions being free of chaotropic salts, and eluting buffer being free of chaotropic salts, as defined in claims 1 to 7.

## Revendications

1. Procédé d'isolement d'acides nucléiques à partir d'un échantillon biologique, comprenant
(a) la digestion de l'échantillon biologique par lyse mécanique et/ou enzymatique et en utilisant une solution de lyse exempte de sel chaotropique,
(b) l'ajout d'une solution de liaison exempte de sel chaotropique, comprenant au moins un solvant organique qui est choisi parmi les carbonates d'alkylène cycliques en C₃₋₄, les diacétates d'alkylèneglycol en C₂₋₃ et leurs dérivés, et au moins un tensioactif non ionique, et des particules superparamagnétiques carboxylées présentant une taille de particules de 0,5 à 10 µm en tant que phase solide liant les acides nucléiques à l'échantillon digéré, et la séparation de la phase solide avec les acides nucléiques liés à celle-ci,
(c) un ou plusieurs lavages de la phase solide séparée avec les acides nucléiques qui y sont liés avec des solutions de lavage identiques ou différentes exemptes de sel chaotropique, et
(d) la désorption des acides nucléiques de la phase solide par ajout d'un tampon d'élution aqueux exempt de sel chaotropique.

2. Procédé selon la revendication 1, dans lequel la solution de lyse exempte de sel chaotropique contient de faibles concentrations d'EDTA et/ou d'un tensioactif anionique.

3. Procédé selon la revendication 1 ou 2, dans lequel, dans la solution de liaison exempte de sel chaotropique, le carbonate d'alkylène cyclique en C₃₋₄ est choisi parmi le carbonate de butylène, le carbonate de propylène et les dérivés de ceux-ci, le diacétate d'alkylèneglycol en C₂₋₃ est choisi parmi le diacétate d'éthylèneglycol, le diacétate de propylèneglycol et les dérivés de ceux-ci, et de préférence la solution de liaison contient au moins un composé qui est choisi parmi le carbonate de butylène, le carbonate de propylène et le diacétate d'éthylèneglycol.

4. Procédé selon une ou plusieurs des revendications 1 à 3, dans lequel, dans la solution de liaison exempte de sel chaotropique,
(i) le tensioactif non ionique, au moins au nombre de un, est choisi parmi des dérivés polyoxyéthylène de monolauréate de sorbitane, de monopalmitate de sorbitane et de monooléate de sorbitane, et des dérivés polyoxyéthylène d'alcools gras, le polyoxyéthylène(20)-monolauréate de sorbitane étant particulièrement préféré ; et/ou
(ii) le rapport (v/v) entre solvant organique et tensioactif non ionique est de 80:20 à 50:50, de préférence de 75:25 à 55:45, et de manière particulièrement préférée d'environ 70:30.

5. Procédé selon une ou plusieurs des revendications 1 à 4, dans lequel, dans l'étape (b),
(i) la quantité de solution de liaison exempte de sel chaotropique ajoutée par rapport au volume de l'échantillon digéré est de 10:1 à 1:10, de préférence de 3:1 à 1:3, et de manière particulièrement préférée d'environ 2:1 à 1:1 (v/v) ; et/ou
(ii) la quantité de phase solide ajoutée par rapport au volume de l'échantillon digéré est de 10:1 à 50:1, de préférence de 20:1 à 40:1, et de manière particulièrement préférée d'environ 25:1 (v/v).

6. Procédé selon une ou plusieurs des revendications 1 à 5, dans lequel les solutions de lavage exemptes de sel chaotropique de l'étape (c) sont des solutions aqueuses contenant du sel et/ou de l'éthanol ou des solutions éthanoliques contenant du sel, de préférence des solutions aqueuses exemptes de sel chaotropique, des mélanges d'éthanol et d'eau exempts de sel chaotropique ou des solutions éthanoliques exemptes de sel chaotropique.

7. Procédé selon une ou plusieurs des revendications 1 à 6, dans lequel le tampon d'élution exempt de sel chaotropique de l'étape (d) est un tampon alcalin à faible teneur en sel.

8. Solution de liaison exempte de sel chaotropique destinée à isoler des acides nucléiques à partir d'un échantillon biologique au moyen de particules superparamagnétiques carboxylées présentant une taille de particules de 0,5 à 10 µm selon le procédé selon une ou plusieurs des revendications 1 à 7, dans laquelle la solution de liaison comprend au moins un solvant organique qui est choisi parmi les carbonates d'alkylène cycliques C₃₋₄, les diacétates d'alkylèneglycol C₂₋₃ et les dérivés de ceux-ci, et au moins un tensioactif non ionique.

9. Solution de liaison exempte de sel chaotropique selon la revendication 8, qui est une solution de liaison exempte de sel chaotropique telle que définie dans la revendication 4.

10. Solution de liaison exempte de sel chaotropique selon la revendication 8 ou 9, qui comprend en outre des particules superparamagnétiques carboxylées présentant une taille de particules de 0,5 à 10 µm en tant que phase solide de liaison d'acides nucléiques.

11. Kit destiné à isoler des acides nucléiques à partir d'un échantillon biologique au moyen de particules superparamagnétiques carboxylées présentant une taille de particules de 0,5 à 10 µm selon le procédé selon une ou plusieurs des revendications 1 à 7, dans lequel le kit comprend une solution de liaison exempte de sel chaotropique selon une ou plusieurs des revendications 8 à 10.

12. Kit selon la revendication 11, comprenant en outre une ou plusieurs des solutions de lyse exemptes de sel chaotropique, des solutions de lavage exemptes de sel chaotropique et du tampon d'élution exempt de sel chaotropique, tels que définis dans les revendications 1 à 7.
